(19) Europäisches Patentamt
European Patent Office
Office européen des brevets

(11) EP 0 580 968 B1

(12) EUROPEAN PATENT SPECIFICATION

(45) Date of publication and mention of the grant of the patent:
**28.08.1996 Bulletin 1996/35**

(51) Int. Cl.[6]: **C07C 401/00**, A61K 31/59

(21) Application number: **93107402.5**

(22) Date of filing: **07.05.1993**

(54) **Vitamin D3 fluorinated analogs**

Fluorierte Analoge Vitamins D3

Derivés fluorinés de la vitamine D3

(84) Designated Contracting States:
**AT BE CH DE DK ES FR GB GR IE IT LI LU MC NL PT SE**

(30) Priority: **20.05.1992 CH 1619/92**
**07.10.1992 US 957500**
**05.11.1992 US 971788**

(43) Date of publication of application:
**02.02.1994 Bulletin 1994/05**

(73) Proprietor: **F. HOFFMANN-LA ROCHE AG**
**4002 Basel (CH)**

(72) Inventors:
- **Baggiolini, Enrico Giuseppe**
**Bloomfield, N.J. 07003 (US)**
- **Shiuey, Shian-Jan**
**Nutley, N.J. 07110 (US)**
- **Uskokovic, Milan Radoje**
**Upper Montclair,N.J. 07043 (US)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**EP-A- 0 325 279**
**EP-A- 0 326 875**
**EP-A- 0 529 528**

- **BLOOD vol. 78, no. 1 , 1 July 1991 , WASHINGTON, US pages 75 - 82 J. ZHOU ET AL 'Development of a novel 1,25(OH)2-vitamin D3 analog with potent ability to induce HL-60 cell differentiation without modulating calcium metabolism.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 580 968 B1

## Description

The invention relates to compounds of the formula

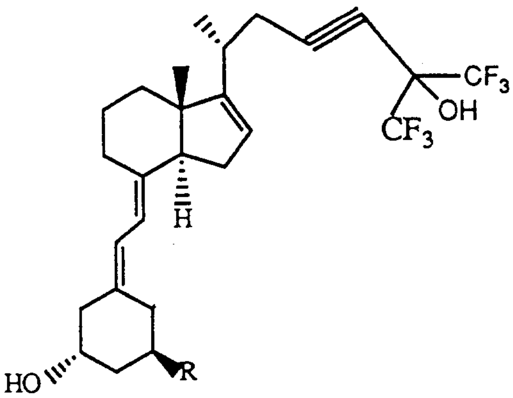

I

wherein R is hydrogen or particularly hydroxy or fluorine.

The invention further relates to the above compounds for use as therapeutically active agents and to the use of said compounds for the manufacture of medicaments for the treatment of hyperproliferative sin diseases, such as psoriasis, for the treatment and prevention of leukemia and cancer, and for the treatment of sebaceous gland diseases, such as acne and seborrheic dermatitis.

The invention also relates to a composition comprising an effective amount of a compound of formula I, or of a mixture of two or more compounds of formula I.

Compounds of formula I as described above stimulate differentiation and decrease proliferation of human keratinocytes. Accordingly, compounds of formula I as described above are useful as agents in the treatment of hyperproliferative skin disorders such as psoriasis, basal cell carcinomas, disorders of keratinization, and keratosis. The compounds of formula I are also useful as agents in the prevention and treatment of leukemia and cancer. The compounds of formula I are also useful for the treatment of sebaceous gland diseases, such as, acne and seborrheic dermatitis.

In the compound of formula I, R is preferably hydroxy or fluorine. A preferred compound of formula I is:

26,26,26,27,27,27-hexafluoro-1α,25-dihydroxy-16-ene-23-yne-19-nor-cholecalciferol.

The compounds of formula I are prepared as hereafter described, with particular reference to the Formula Schemes below,

SCHEME I

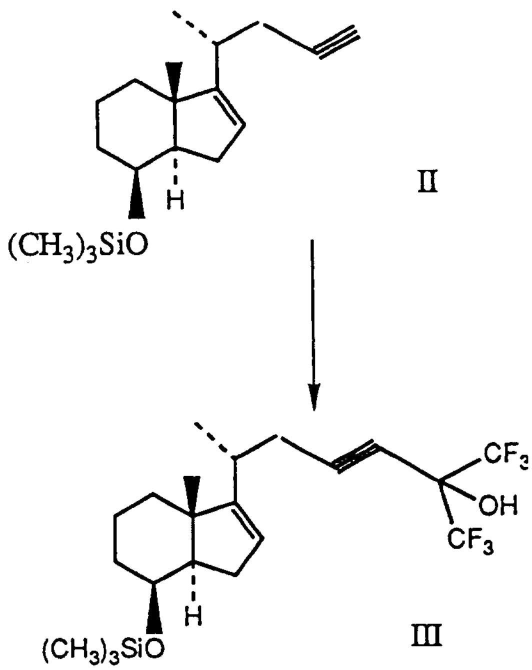

II

III

In above Formula Scheme I, the compound of formula II, described in EP0325279, is converted to the compound of formula III by reaction with a base, such as, n-butyllithium, and hexafluoroacetone. The reaction is conducted in an ether solvent such as tetrahydrofuran at about -50°C to about -100°C. The compound of formula III is recovered by quenching the reaction, followed by a conventional work-up and a purification, e.g. by chromatography.

The compound of formula V is reacted with n-butyllithium and the compound of formula VI, preferably in a mixture of hexane and tetrahydrofuran at a temperature of about -75°C to give a compound of formula I after removal of silyl protecting groups, conveniently with tetrabutylammonium fluoride in tetrahydrofuran solvent.

SCHEME II

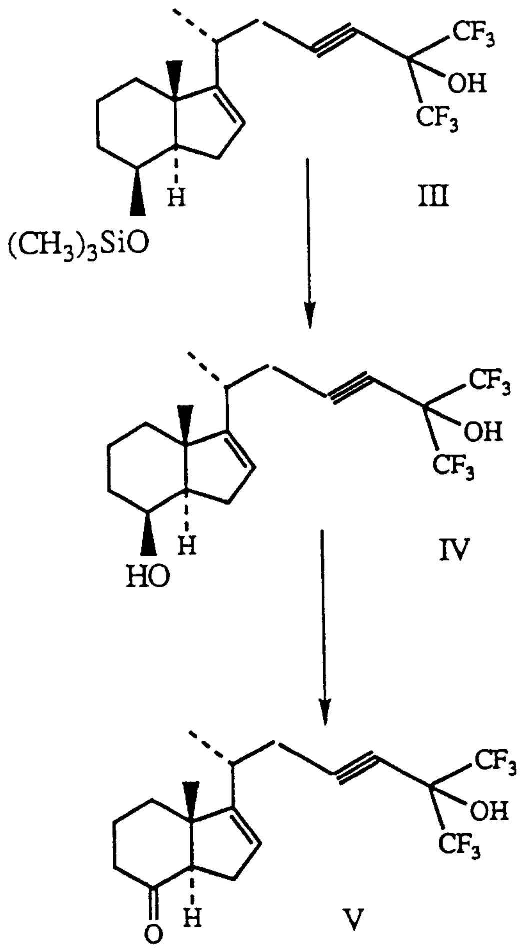

III

IV

V

In reaction Scheme II, the compound of formula III is deprotected to give the compound of formula IV by reaction with tetrabutylammonium fluoride in an ether solvent such as, tetrahydrofuran. The compound of formula IV is reacted with pyridinium chlorochromate in a chlorinated hydrocarbon solvent such as methylene chloride at room temperature to give the compound of formula V. The compound of formula I is prepared by a process which comprises reacting the compound of formula

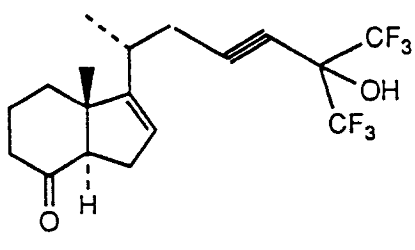

V

with n-butyllithium and a compound of formula

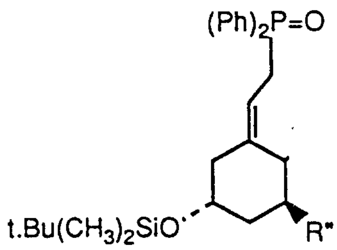

VI

wherein R" is H, OH, F or $OSi(CH_3)_2$ t.Bu and t.Bu is tert-butyl and Ph is phenyl,
in a solvent at low temperature to give the compound of formula I after removal of the silyl protecting group(s).

The compounds of formula VI are known [when R" is $OSi(CH_3)_2$ tert-butyl: see Tetrahedron Letters Vol. 32 No. 52 (1991) 7663-6] or can be prepared according to known methods.

The compounds of formula I can be administered orally, for the treatment of neoplastic diseases such as leukemia, to warmblooded animals which need such treatment, e.g. to an adult human in dosages that are in the range of about 0.1 to 10 µg per day. They can also be administered topically or orally for the treatment of hyperproliferative skin diseases such as psoriasis, basal cell carcinomas, disorders of keratinization, and keratosis, to warmblooded animals which need such treatment, e.g. to an adult human in dosages that are in the range of about 0.01 to 100 µg per gram of topical formulation per day, or in the range of about 0.001 to 100 µg orally per day. They can further be administered topically or orally for the treatment of sebaceous gland diseases such as acne and seborrheic dermatitis to a host requiring such treatment, e.g. in dosages that are in the range of about 0.1 to about 1000 µg per gram of topical formulation per day, or orally to an adult human in dosages that are in the range of about 0.07 µg to 770 µg per day, more preferably in the range of about 0.7 µg to 70 µg per day.

The useful activity of compounds of formula I as agents for the treatment of neoplastic diseases, such as leukemia, can be demonstrated by the following test procedures.

**HL-60 Differentiation Assay:**

The HL-60 tumor cell line was originally derived from a patient with promyelocytic leukemia. The cells are maintained in suspension and diluted to 200,000 cells/ml in 5 ml of suspension media. Compounds were tested at four concentrations in duplicate flasks. Control flasks were supplemented with vehicle alone at the highest concentration such as 0.1% ethanol. Flasks are incubated upright for 4 days in 5% $CO_2$ at 37°C. On day 4, a 1 ml aliquot of cells was centrifuged, the media removed and the cells resuspended in a 0.2 ml of solution of nitroblue tetrazolium/phorbol 12 myristate 13-acetate (NBT/TPA). The cells are suspended and incubated at 37°C for 30 min. prior to transferring to ice. An aliquot is removed and a total of 200 cells is counted using a hemocytometer. Cells without pigmented granules are judged to be undifferentiated while those containing blue black formazan (indicating conversion of NBT) granules were scored as differentiated. Results are expressed as percent differentiated cells by calculating the ratio of the number of dark cells per total number of cells counted. The results are set forth below.

| Compound | % Differentiated cells | | | | | $ED_{50}$ |
|---|---|---|---|---|---|---|
| | $10^{-10}$ | $10^{-9}$ | $10^{-8}$ | $10^{-7}$ | $10^{-6}$ | |
| A | | 64 | 89 | 91 | 90 | < 1 nM |
| B | NT | 15 | 18 | 72 | 88 | 35 nM |
| C | NT | 61 | 90 | 93 | 96 | < 1 nM |
| D | 1.5 | 56.5 | 89.5 | NT | NT | 0.8 nM |
| NT means not tested. | | | | | | |

Compound D is: 1,25-dihydroxy-16-ene-23-yne-26,27-hexafluoro-19-nor-cholecalciferol, a compound of the instant invention.

Compounds A, B and C are described in Blood 78 (1991) 75-82. In contradinstinction to these compounds, those of the instant formula I are 19-nor-cholecalciferol derivatives.

Oral dosage forms comprising compounds of formula I of the invention may be incorporated in capsules, tablets and the like with pharmaceutically acceptable carrier materials. Illustrative of such acceptable carrier materials are the following: a binder such as gum tragacanth, acacia, corn starch, or gelatin; an excipient such as dicalcium phosphate, a disintegrating agent such as corn starch, potato starch, or algenic acid; a lubricant such as magnesium stearate, a sweetening agent such as sucrose, lactose, or saccharin; a flavoring agent such as peppermint, oil of wintergreen or cherry. Various other materials may be present as coating or to otherwise modify the physical form of the dosage unit. For instance, tablets may be coated with shellac, sugar, or both. A syrup or elixir may contain the active compound, sucrose as a sweetening agent, methyl and propyl parabens as preservatives, a dye, and a flavoring such as cherry or orange flavor.

The topical dosage forms comprise gels, creams, lotions, ointments, powders, aerosols and other conventional forms for applying medication at the site of inflammation, i.e. to the skin or to mucous membranes, for example to the mucous lining of the mouth or lower colon. Ointments and creams encompassing formulations having oleaginous, adsorbable, water-soluble and emulsion-type bases such as petrolatum, lanolin or polyethylene glycols. Lotions vary from simple solutions to aqueous or hydroalcoholic preparations containing finely divided substances. Lotions can contain suspending or dispersing agents, for example, cellulose derivatives such as ethyl or methyl cellulose; gelatin or gums, which incorporate the active ingredient in a vehicle made up of water, alcohol or glycerin. Gels are made by gelling a solution or suspension-of the active ingredient in a carrier vehicle. The vehicles, which can be hydrous or anhydrous, are gelled using e.g. carboxy polymethylene, and neutralized to a proper gel consistency with the use of alkalies, such as, sodium hydroxide and amines, such as, polyethylenecocoamine.

## REFERENCE EXAMPLE 1

To a solution of 273 mg of [5S-Z]-2-[2-[2-methylene-5[[(1,1-dimethylethyl)dimethylsilyl]oxy]cyclohexylidene]ethyl] diphenyl phosphine oxide in 6.4 ml anhydrous tetrahydrofuran. To this was added, at -75°C, 0.343 ml of 1.6 M n-butyl lithium in hexane dropwise under argon, until red color of the reaction mixture developed. After stirring for 6 min, a solution of 83 mg of [3aR-[1(R*),3aα,7aβ]]-3,3a,5,6,7,7a-hexahydro-7a-methyl-1-[6,6,6-trifluoro-5-hydroxy-1-methyl-5-(trifluoromethyl)-3-hexynyl]-4H-inden-4-one in 6.5 ml of anhydrous tetrahydrofuran was added dropwise. This reaction mixture was stirred in dark, and then quenched with 2.7 ml of a 1:1 mixture of 2N Rochelle salt and 2M $KHCO_3$ at -75°C, and allowed to warm up to room temperature. It was diluted with 10.5 ml of the same salt mixture and extracted with ethyl acetate. The extract was washed with saturated brine, dried and evaporated. The crude product was purified by flash chromatography on silica gel with ethyl acetate-hexane 1:5 to give 78 mg of silyl protected 25-hydroxy-16-ene-23-yne-26,27-hexafluoro-cholecalciferol.

To a solution of 76 mg of the silyl intermediate in 3.5 ml anhydrous tetrahydrofuran was added 0.70 ml of 1M solution of tetrabutyl ammonium fluoride in tetrahydrofuran, and the reaction mixture was stirred at room temperature for 16 hrs. It was then quenched with 2.5 ml of half-saturated $NaHCO_3$ and stirred at room temperature. The mixture was extracted with ethyl acetate, and the extract was washed with half-saturated $NaHCO_3$ and brine, dried and evaporated. The crude product was purified by flash chromatography with ethyl acetate-hexane 1:1 to give 49 mg of 25-hydroxy-16-ene-23-yne-26,27-hexafluoro-cholecalciferol; $[\alpha]_D^{25}$ = +77.3° (c 0.15, $CH_3OH$).

## REFERENCE EXAMPLE 2

To a solution of 159 mg of [3S-(3α,5β,Z)]-2-[2-[2-methylene-3-fluoro-5-[[(1,1-dimethylethyl)dimethylsilyl]oxy]cyclohexylidene]ethyl]diphenyl phosphine oxide in 4.3 ml anhydrous tetrahydrofuran was added, at -75°C, 0.201 ml of 1.6 M n-butyllithium in hexane under argon, until red color of the reaction mixture developed. After stirring for 6 min, a solution of 47 mg of [3aR-[1(R*),3aα, 7aβ]]-3,3a,5,6,7,7a-hexahydro-7a-methyl-1-[6,6,6-trifluoro-5-hydroxy-1-methyl-5-(trifluoromethyl)-3-hexynyl]-4H-inden-4-one in 2.5 ml of anhydrous tetrahydrofuran was added dropwise. This reaction mixture was stirred in dark, and then quenched with a 1:1 mixture of 2N Rochelle salt and 2M $KHCO_3$ at -75°C, and allowed to warm up to room temperature. It was diluted with 10.5 ml of the same salt mixture and extracted with ethyl acetate. The extract was washed with saturated brine, dried and evaporated. The crude product was purified by flash chromatography on silica gel with ethyl acetate-hexane 1:5 to give 27 mg of silyl protected 1α-fluoro-25-hydroxy-16-ene-23-yne-26,27-hexafluoro-cholecalciferol.

To a solution of 27 mg of the silyl intermediate in 1.8 ml anhydrous tetrahydrofuran was added 0.257 ml of 1M solution of tetrabutyl ammonium fluoride in tetrahydrofuran, and the reaction mixture was stirred at room temperature. It was then quenched with half-saturated $NaHCO_3$ and stirred at room temperature. The mixture was extracted with ethyl acetate, and the extract was washed with half-saturated $NaHCO_3$ and brine, dried and evaporated. The crude product was purified by flash chromatography with ethyl acetate-hexane 1:1.3 to give 19 mg of 1α-fluoro-25-hydroxy-16-ene-23-yne-26,27-hexafluoro-cholecalciferol as a glass; $[\alpha]_D^{25}$ = +71.7° (c 0.12, $CH_3OH$).

**EXAMPLE 3**

To a solution of 605 mg of [3R-(3α,5β,Z)-3,5-bis[[(1,1-dimethylethyl)dimethylsilyl]oxy]cyclohexylidene]ethyl]diphenyl phosphine oxide in 7 ml anhydrous tetrahydrofuran was added at -75°C, 0.645 ml of 1.6M n-butyllithium in hexane under argon. After stirring, a solution of 162 mg of [3aR-[1(R*),3aα,7aβ]]-3,3a,5, 6,7,7a-hexahydro-7a-methyl-1-[6,6,6-trifluoro-5-hydroxy-methyl-5-(trifluoromethyl)-3-hexynyl]-4H-inden-4-one in 4.5 ml anhydrous tetrahydrofuran was added. The reaction mixture was stirred in dark at -75°C, and then quenched with 2.6ml of 1:1 mixture of 2N Rochelle salt and 2N $KHCO_3$ solution and was allowed to warm to room temperature. It was then diluted with 10 ml of the same salt solution and extracted with ethyl acetate. The extract was washed with brine, dried and evaporated. The crude product was purified by flash chromatography on silica gel column with ethyl acetate-hexane 1:8 to give 168 mg of the disilyl-protected 1,25-dihydroxy-16-ene-23-yne-26,27-hexafluoro-19-nor-cholecalciferol.

To 168 mg of the disilyl protected compound in 4.5 ml anhydrous tetrahydrofuran there was added 2.7 ml of 1 M tetrabutylammonium fluoride in tetrahydrofuran, and the mixture was stirred for 43 hrs under argon. The reaction was then quenched with 5 ml of water and stirred at room temperature. It was then extracted with ethyl acetate. The extract was washed with brine, dried and evaporated. The product was purified by flash chromatography with ethyl-acetate-hexane 3:1 to give 99 mg of 1,25-dihydroxy-16-ene-23-yne-26,27-hexafluoro-19-nor-cholecalciferol as foam: $[\alpha]_D^{25}$ = +25°C (c 0.2 ethanol).

**EXAMPLE 4**

In a manner analogous to that in Reference Example 1, by using [5S-Z]-5-[[(1,1-dimethylethyl)dimethylsilyl]oxy]cyclohexylidene]ethyl]diphenyl phosphine oxide instead of [5S-Z]-2-[2-[2-methylene-5-[[(1,1-dimethylethyl)dimethylsilyl]oxy]cyclohexylidene]ethyl]diphenyl phosphine oxide, there is obtained 25-hydroxy-16-ene-23-yne-26,27-hexafluoro-19-nor-cholecalciferol.

**EXAMPLE 5**

In a manner analogous to that in Reference Example 3, by using [3R-(3α,5β,Z)]-3-fluoro-5[[(1,1-dimethylethyl)dimethylsilyl]oxy]cyclohexylidene]ethyl]diphenyl phosphine oxide instead of [3S-(3α,5β,Z)]-2-[2-[2-methylene-3-fluoro-5[[(1,1-dimethylethyl)dimethylsilyl]oxy]cyclohexylidene]ethyl]diphenyl phosphine oxide, there is obtained 1α-fluoro-25-hydroxy-16-ene-23-yne-26,27-hexafluoro-19-nor-cholecalciferol.

The following pharmaceutical compositions were prepared in a manner known per se:

**EXAMPLE A**

| Oral Dosage Form Soft Gelatin Capsule | mg/Capsule |
|---|---|
| Compound B | 0.0001-0.010 |
| Butylated Hydroxytoluene (BHT) | 0.016 |
| Butylated Hydroxyanisole (BHA) | 0.016 |
| Fractionated Coconut Oil (Neobee M-5) | 160.0 |

**EXAMPLE B**

| Topical Cream | mg/g |
|---|---|
| Compound B | 0.001-1.0 |
| Cetyl Alcohol | 1.5 |
| Stearyl Alcohol | 2.5 |
| Span 60 (Sorbitan monostearate) | 2.0 |
| Arlacel 165 (Glyceryl monostearate and polyoxyethylene glycol stearate blend) | 4.0 |
| Tween 60 (polysorbate 60) | 1.0 |
| Mineral Oil | 4.0 |
| Propylene Glycol | 5.0 |
| Propylparaben | 0.05 |
| BHA | 0.05 |
| Sorbitol Solution | 2.0 |
| Edetate Disodium | 0.01 |
| Methylparaben | 0.18 |
| Distilled Water | q.s. to 100 g |

## Claims

1. A compound of the formula

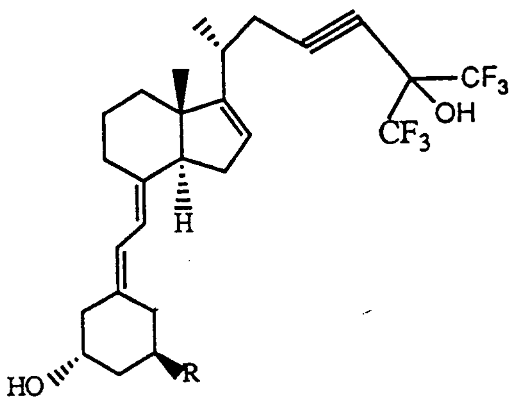

I

wherein R is hydrogen or particularly hydroxy or fluorine.

2. The compound in accordance with Claim 1:
26,26,26,27,27,27-hexafluoro-1α,25-dihydroxy-16-ene-23-yne-19-nor-cholecalciferol.

3. A compound according to Claim 1 or 2 for use as a therapeutically active agent, particularly for the treatment of hyperproliferative disorders of the skin, such as psoriasis, for the treatment of cancer and leukemia, and for the treatment of sebaceous gland diseases, such as acne and seborrheic dermatitis.

4. A pharmaceutical composition, particularly for the treatment of hyperproliferative disorders of the skin, such as psoriasis, for the treatment of cancer and leukemia, and for the treatment of sebaceous gland diseases, such as acne

and seborrheic dermatitis comprising an effective amount of a compound of the formula I according to Claim 1, or of a mixture of two or more compounds of formula I, and a pharmaceutically acceptable carrier.

5. The use of a compound as in Claim 1 or 2 for the manufacture of a medicament for the treatment of hyperproliferative disorders of the skin, such as psoriasis, for the treatment of cancer and leukemia, and for the treatment of sebaceous gland diseases, such as acne and seborrheic dermatitis.

**Patentansprüche**

1. Eine Verbindung der Formel

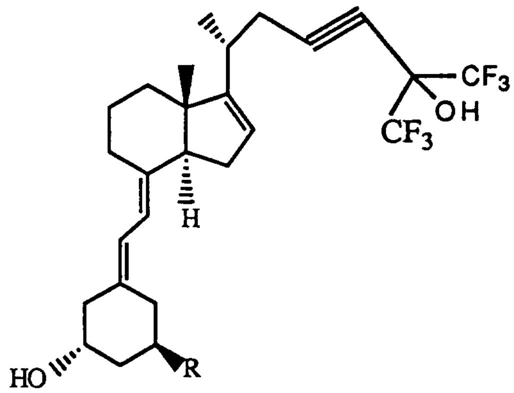

I

worin R Wasserstoff oder insbesondere Hydroxy oder Fluor ist.

2. Die Verbindung nach Anspruch 1:
26, 26, 26, 27, 27, 27-Hexafluoro-1$\alpha$, 25-dihydroxy-16-en-23-yn-19-nor-cholecalciferol.

3. Eine Verbindung nach Anspruch 1 oder 2 zur Verwendung als therapeutisch wirksames Mittel, insbesondere für die Behandlung von hyperproliferativen Hautkrankheiten, wie Psoriasis, für die Behandlung von Krebs und Leukämie und für die Behandlung von Krankheiten der Talgdrüsen, wie Akne und seborrhoische Dermatitis.

4. Pharmazeutisches Präparat, insbesondere für die Behandlung von hyperproliferativen Hautkrankneiten, wie Psoriasis, für die Behandlung von Krebs und Leukämie und für die Behandlung von Krankheiten der Talgdrüsen, wie Akne und seborrhoische Dermatitis, bestehend aus einer wirksamen Menge einer Verbindung der Formel I nach Anspruch 1 oder einem Gemisch von zwei oder mehr Verbindungen der Formel I und einem pharmazeutisch annehmbaren Trägermaterial.

5. Die Verwendung einer Verbindung nach Anspruch 1 oder 2 für die Herstellung eines Arztneimittels für die Behandlung von hyperproliferativen Hautkrankheiten, wie Psoriasis, für die Behandlung von Krebs und Leukämie und für die Behandlung von Krankheiten der Talgdrüsen, wie Akne und seborrhoische Dermatitis.

**Revendications**

1. Composé de formule

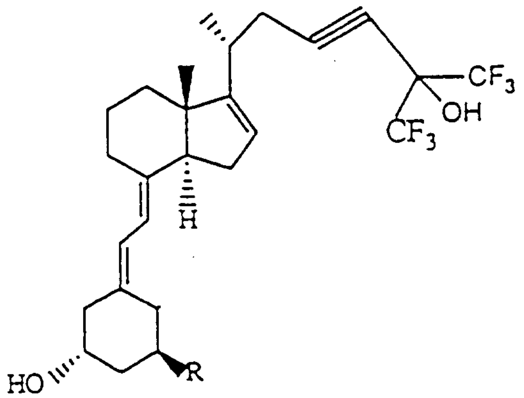

I

dans laquelle R représente un atome d'hydrogène ou en particulier un groupe hydroxy ou un atome de fluor.

2. Composé selon la revendication 1 qui est le 26,26,26,27,27,27-hexafluoro-1$\alpha$,25-dihydroxy-16-ène-23-yne-19-nor-cholicalciférol.

3. Composé selon la revendication 1 ou 2, à utiliser comme agent thérapeutiquement actif, en particulier pour le traitement des troubles hyperprolifératifs de la peau tels que le psoriasis, pour le traitement du cancer et de la leucémie et pour le traitement des maladies des glandes sébacées comme l'acné et l'eczéma séborrhéique.

4. Composition pharmaceutique, en particulier pour le traitement des troubles hyperprolifératifs de la peau, comme le psoriasis, pour le traitement du cancer et de la leucémie et pour le traitement des maladies des glandes sébacées, comme l'acné et l'eczéma séborrhéique, qui comprend une quantité efficace d'un composé de formule I selon la revendication 1, ou d'un mélange de deux composés ou plus de formule I, et un véhicule pharmaceutiquement acceptable.

5. Utilisation d'un composé selon la revendication 1 ou 2, pour la préparation d'un médicament pour le traitement des troubles hyperprolifératifs de la peau, comme le psoriasis, pour le traitement du cancer et de la leucémie et pour le traitement des maladies des glandes sébacées, comme l'acné et l'eczéma séborrhéique.